(19) Europäisches Patentamt
European Patent Office
Office européen des brevets

(11) **EP 3 274 012 B1**

(12) **EUROPÄISCHE PATENTSCHRIFT**

(45) Veröffentlichungstag und Bekanntmachung des Hinweises auf die Patenterteilung:
**21.07.2021 Patentblatt 2021/29**

(21) Anmeldenummer: **16710772.1**

(22) Anmeldetag: **21.03.2016**

(51) Int Cl.:
**A61M 1/16** (2006.01)

(86) Internationale Anmeldenummer:
**PCT/EP2016/056151**

(87) Internationale Veröffentlichungsnummer:
**WO 2016/150915 (29.09.2016 Gazette 2016/39)**

(54) **TEMPERATURSTÖRUNGSUNABHÄNGIGE BILANZIERUNGSEINRICHTUNG UND BILANZIERUNGSVERFAHREN**

TEMPERATURE VARIATION-INDEPENDENT BALANCING APPARATUS AND BALANCING METHOD

DISPOSITIF D'ÉTABLISSEMENT DE BILAN INDÉPENDANT DES PERBURBATIONS DE TEMPÉRATURE, ET PROCÉDÉ D'ÉTABLISSEMENT DE BILAN

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **24.03.2015 DE 102015104430**

(43) Veröffentlichungstag der Anmeldung:
**31.01.2018 Patentblatt 2018/05**

(73) Patentinhaber: **Fresenius Medical Care Deutschland GmbH**
**61352 Bad Homburg (DE)**

(72) Erfinder:
• **ABEL, Petra**
**61169 Friedberg (DE)**
• **HEIDE, Alexander**
**65817 Eppstein (DE)**
• **NIKOLIC, Dejan**
**65824 Schwalbach am Taunus (DE)**
• **PETERS, Arne**
**61352 Bad Homburg (DE)**
• **SCHULTE, Elke**
**97422 Schweinfurt (DE)**
• **WIKTOR, Christoph**
**63571 Gelnhausen (DE)**

(74) Vertreter: **Bobbert & Partner Patentanwälte PartmbB Postfach 1252 85422 Erding (DE)**

(56) Entgegenhaltungen:
**EP-A1- 0 715 859       EP-A1- 2 497 507**
**EP-A1- 2 532 999       DE-A1-102010 003 642**
**DE-A1-102010 031 802   US-A1- 2010 038 322**

**Beschreibung**

Die vorliegende Erfindung betrifft eine medizinische Funktionseinrichtung gemäß Anspruch 1, eine Bilanzierungseinrichtung gemäß Anspruch 11 und eine medizinische Behandlungsvorrichtung gemäß Anspruch 14.

In der Medizin, insbesondere im Bereich der Blutbehandlung, beispielsweise der Dialyse, werden dem Patienten Flüssigkeiten zugeführt und/oder dem Patienten Flüssigkeiten entzogen. Die genaue Bilanzierung dieser dem Patienten zugeführten bzw. entzogenen Flüssigkeitsmengen ist von besonderer Bedeutung für die Sicherheit und Gesundheit des Patienten.

Zur Bilanzierung werden in der Praxis Bilanziereinrichtungen verwendet. Diese können mit Blutbehandlungsvorrichtungen, beispielsweise Dialysevorrichtungen, verbunden sein (siehe z.B. EP0715859).

Eine Aufgabe der vorliegenden Erfindung ist es, eine weitere Bilanzierungseinrichtung und ein weiteres Bilanzierungsverfahren zur Bilanzierung von Flüssigkeitsströmen anzugeben. Ferner sollen eine medizinische Funktionseinrichtung sowie eine medizinische Behandlungsvorrichtung angegeben werden.

Alle oder manche der mit der erfindungsgemäßen Funktionseinrichtung erzielbaren Vorteile lassen sich ungeschmälert auch mit der erfindungsgemäßen Bilanzierungseinrichtung sowie mit der medizinischen Behandlungsvorrichtung erzielen.

Die erfindungsgemäße Aufgabe wird durch Vorrichtungen bzw. Verfahren mit den Merkmalen der Ansprüche 1, 11 und 14 gelöst.

Erfindungsgemäß wird somit eine Funktionseinrichtung, welche vorzugsweise eine medizinische Funktionseinrichtung ist, vorgeschlagen. Sie umfasst wenigstens einen Fluidkreislauf, oder wenigstens einen Abschnitt hiervon, oder ist hiermit verbunden.

Zudem weist die Funktionseinrichtung und/oder der Fluidkreislauf wenigstens eine Wärmeaustauscheinrichtung auf oder ist hiermit verbunden. Die Wärmeaustauscheinrichtung ist dabei wenigstens zwischen zwei unterschiedlichen Abschnitten des Fluidkreislaufs, insbesondere zwischen dem ersten Abschnitt und dem zweiten Abschnitt, angeordnet und/oder ausgebildet.

Die erfindungsgemäße Bilanzierungseinrichtung ist vorgesehen und ausgebildet und/oder konfiguriert zum Bestimmen wenigstens einer Fluidbilanz, insbesondere zwischen wenigstens oder genau einem ersten Volumenstrom in einem ersten Abschnitt eines Fluidkreislaufs und wenigstens einem zweiten Volumenstrom in einem zweiten Abschnitt des Fluidkreislaufs. Die Bilanzierungseinrichtung weist dabei eine erfindungsgemäße Funktionseinrichtung auf und/oder kann in Datenverbindung mit wenigstens einem Teil der Funktionseinrichtung stehen, insbesondere mit wenigstens einem Durchflusssensor und/oder wenigstens einer Pumpe, beispielsweise einer Blutpumpe, und/oder ist verbunden und/oder verbindbar mit einem solchen.

Die erfindungsgemäße medizinische Behandlungsvorrichtung, welche zum Behandeln eines medizinischen Fluids ausgebildet und/oder konfiguriert ist, weist wenigstens eine erfindungsgemäße Bilanzierungseinrichtung und/oder wenigstens eine erfindungsgemäße Funktionseinrichtung auf und/oder ist mit wenigstens einer der beiden, vorzugsweise lösbar, verbunden.

Ein nicht beanspruchtes Verfahren betrifft das Bestimmen wenigstens einer Fluidbilanz zwischen wenigstens einem ersten Volumenstrom, insbesondere eines ersten Abschnitts eines Fluidkreislaufs, und wenigstens einem zweiten Volumenstrom, insbesondere eines zweiten Abschnitts des Fluidkreislaufs.

Das Verfahren betrifft zusätzlich oder alternativ das Steuern und/oder Regeln (beide Begriffe werden im Folgenden der besseren Lesbarkeit halber als "Steuern" bezeichnet, was optional aber auch ein Regeln umfasst) wenigstens eines Volumenstroms wenigstens eines Abschnitts des Fluidkreislaufs der Funktionseinrichtung.

Das Verfahren weist wenigstens den Schritt auf, eine erfindungsgemäße Funktionseinrichtung und/oder Bilanzierungseinrichtung bereitzustellen.

Das Verfahren umfasst wenigstens das Erfassen wenigstens eines Volumenstroms, beispielsweise im ersten oder im zweiten Abschnitt.

Im Verfahren wird wenigstens eine Fluidbilanz aus einem ersten und einem zweiten Volumenstrom gebildet.

Im Verfahren wird wenigstens die Fluidbilanz (hier als FB abgekürzt) zum Steuern und/oder Regeln wenigstens eines Volumenstroms des Fluidkreislaufs, einer Pumprate wenigstens einer Pumpe des Fluidkreislaufs und/oder eines Querschnitts wenigstens eines Abschnitts des Fluidkreislaufs verwendet. Vorteilhafte Weiterbildungen der vorliegenden Erfindung sind jeweils Gegenstand der Unteransprüche.

Erfindungsgemäße Ausführungsformen können einige oder alle der folgenden Merkmale in beliebiger Kombination aufweisen.

Bei allen oben gemachten und bei allen folgenden Ausführungen ist der Gebrauch des Ausdrucks "kann sein" bzw. "kann haben" usw. synonym zu "ist vorzugsweise" bzw. "hat vorzugsweise" usw. zu verstehen und soll erfindungsgemäße Ausführungsformen erläutern.

Erfindungsgemäße Ausführungsformen können manche, einige oder alle der folgenden Merkmale in beliebiger Kombination aufweisen, soweit dies für den Fachmann nicht erkennbar technisch unmöglich ist. Vorteilhafte Weiterbildungen der vorliegenden Erfindung können jeweils auch Gegenstand der Unteransprüche sein.

Wann immer hierin Zahlenworte genannt werden, so versteht der Fachmann diese als Angabe einer zahlenmäßig unteren Grenze. Sofern dies zu keinem für den Fachmann erkennbaren Widerspruch führt, liest der Fachmann daher beispielsweise bei der Angabe "ein" oder "einem" stets "wenigstens ein" oder "wenigstens einem" mit. Dieses Verständnis ist ebenso von der vorliegenden Erfindung mit umfasst wie die Auslegung, dass ein Zahlenwort wie beispielsweise "ein" alternativ als "genau ein" gemeint sein kann, wo immer dies für den Fachmann nicht erkennbar technisch unmöglich ist. Beides ist von der vorliegenden Erfindung umfasst und gilt für alle hierin verwendeten Zahlenworte.

Hierin gemachte räumliche Angaben wie "oben", "unten", usw. beziehen sich im Zweifel auf die Darstellung, wie sie den hier beigefügten Figuren zu entnehmen ist.

Wann immer hierin die Rede von "geeignet", "vorgesehen", "ausgelegt", "konfiguriert" und/oder "programmiert" ist, kann der Fachmann dies als eine besondere Ausgestaltung der betreffenden Vorrichtung verstehen. Die vorgenannten Begriffe können hier austauschbar verwendet werden.

Obgleich die Erfindung im Folgenden vornehmlich anhand der Dialysebehandlung beschrieben wird, ist die Erfindung hierauf nicht beschränkt.

Der Begriff "Volumenstrom", wie er hierin verwendet wird, kann als das Volumen eines Fluids, das sich innerhalb eines gegebenen Zeitraums durch einen Querschnitt, beispielsweise des Fluidkreislaufs, bewegt hat, bezogen auf den hierfür erforderlichen Zeitraum, verstanden werden. Ein Volumenstrom kann beispielsweise in ml pro Minuten oder pro Stunde angegeben werden.

Der Begriff "Massenstrom", wie er hierin verwendet wird, kann gleich der Dichte des Fluids mal dem Volumenstrom sein.

Der Begriff "Erfassen", wie er hierin verwendet wird, kann allgemein ein Messen, ein Ermitteln, ein Lesen eines, beispielsweise gespeicherten und/oder eingestellten Wertes und/oder ein Berechnen, insbesondere aus bekannten, gespeicherten, erfassten und/oder eingestellten Werten, sein. Unter einem "Abschnitt des Fluidkreislaufs" kann in bestimmten, beispielhaften Ausführungsformen insbesondere der erste Abschnitt oder der zweite Abschnitt des Fluidkreislaufs, insbesondere eines Dialysat- und/oder eines Substituatkreislaufs, verstanden werden.

Mit einem "Volumenstrom des Fluidkreislaufs" kann in bestimmten, beispielhaften Ausführungsformen insbesondere der Volumenstrom des ersten Abschnitts oder der Volumenstrom des zweiten Abschnitts des Fluidkreislaufs, insbesondere eines Dialysat- und/oder eines Substituatkreislaufs, verstanden werden.

In erfindungsgemäßen Ausführungsformen weist die, insbesondere medizinische oder medizintechnische, Funktionseinrichtung wenigstens einen Fluidkreislauf, insbesondere einen Prozessfluidkreislauf, beispielsweise einen Flüssigkeitskreislauf, oder Abschnitte desselben auf.

Der Begriff "Fluidkreislauf", wie er hierin verwendet wird, bezeichnet ein Fluidsystem, einen Schlauchsatz, einen Blutschlauchsatz oder dergleichen, jeweils dazu geeignet, vorgesehen und/oder ausgebildet, um Prozessfluide, medizinische Flüssigkeiten, beispielsweise Blut, Substituat, Dialysat und/oder Kombinationen hiervon, aufzunehmen und von diesen durchströmt zu werden. Die Prozessfluide können zum Zwecke eines Prozesses wie Spülen, Primen, Substituieren, Senken von Schadstoffkonzentrationen und dergleichen zum Einsatz kommen. Ein Fluidkreislauf muss keinen geschlossenen Kreislauf ermöglichen. Ein Fluidkreislauf kann wenigstens eine Filtereinrichtung, beispielsweise einen Blutfilter, und/oder wenigstens eine Pumpe, beispielsweise eine Blut- oder Substituatpumpe, aufweisen.

In einigen erfindungsgemäßen, beispielhaften Ausführungsformen ist der Fluidkreislauf oder wenigstens ein Abschnitt desselben vorgesehen und/oder ausgebildet zum Aufnehmen und/oder Fördern wenigstens eines Fluids, insbesondere eines medizinischen Fluids.

In einigen erfindungsgemäßen, beispielhaften Ausführungsformen weist die Funktionseinrichtung wenigstens einen Kanal auf, welcher wenigstens dazu geeignet und/oder ausgebildet ist, eine Flüssigkeit zu befördern und/oder zu führen.

In einigen erfindungsgemäßen, beispielhaften Ausführungsformen ist der Fluidkreislauf ein, insbesondere arterieller und/oder venöser, Blut-, Dialysat- und/oder Substituatkreislauf.

Die Funktionseinrichtung kann ferner wenigstens eine Filtereinrichtung aufweisen, welche innerhalb des Fluidkreislaufs, insbesondere zwischen einem Blutkreislauf und wenigstens einem Dialysatkreislauf, oder wenigstens eines Abschnitts hiervon, angeordnet ist. Die Filtereinrichtung kann ein Blutfilter oder Dialysator sein.

In manchen erfindungsgemäßen, beispielhaften Ausführungsformen ist die Wärmeaustauscheinrichtung derart dimensioniert, angeordnet, verbunden und/oder ausgebildet, dass die Temperatur des ersten Abschnitts und/oder insbesondere des den ersten Abschnitt des Fluidkreislaufs durchströmenden Fluids und die Temperatur des zweiten Abschnitts, insbesondere des den zweiten Abschnitt des Fluidkreislaufs durchströmenden Fluids, innerhalb einer vorbestimmbaren, vordefinierten, einstellbaren und/oder vordefinierbaren, wenigstens begrenzten oder vorbestimmten, Zeit an- oder ausgeglichen, oder die Temperaturen im Wesentlichen gleich oder identisch sind.

Alternativ oder ergänzend kann in manchen erfindungsgemäßen, beispielhaften Ausführungsformen die Temperaturdifferenz der in den betreffenden Abschnitten durchströmenden Fluide und/oder der jeweiligen Abschnitte vor und nach dem Verlassen der Wärmeaustauscheinrichtung wenigstens reduziert sein, beispielsweise um wenigstens 10%, 20%, 30%, 40%, 50%, 60% oder mehr.

Der Begriff "ausgleichen oder gleich", wie er hierin verwendet wird, kann allgemein so verstanden werden, dass die Temperaturen der betroffen Abschnitte und/oder der in den betreffenden Abschnitten vorliegenden oder durchströmenden Fluide nach Verlassen der Wärmetauscheinrichtung gleich oder im Wesentlichen gleich sind. Dabei kann insbesondere die Temperaturdifferenz der in den betreffenden Abschnitten durchströmenden Fluide vor und nach dem Verlassen der Wärmetauscheinrichtung reduziert und/oder angeglichen werden. Insbesondere kann die Differenz der ausgehenden Temperaturen, geringer, beispielsweise deutlich geringer, als die Differenz der eingehenden oder ursprünglichen Temperaturen sein.

Die Temperaturen können beispielsweise ab einer Differenz kleiner oder gleich 10°C als "im Wesentlichen" gleich angesehen werden. Vorzugsweise sind Temperaturdifferenzen von 2°C oder geringer, insbesondere von deutlich kleiner als 1°C zu erzielen. Der Fachmann erkennt, dass diese Angabe von der maximalen Temperaturdifferenz im Bilanzierkreislauf abhängen kann. Vorzugsweise sollte die maximale Temperaturdifferenz um 30 bis 70% verringert werden.

In einigen erfindungsgemäßen, beispielhaften Ausführungsformen ist der erste Abschnitt in einem Betriebszustand der Funktionseinrichtung, insbesondere in Bezug auf eine Strömungsrichtung des Fluids während des Betriebs der Funktionseinrichtung, beispielsweise während einer Behandlung, einer Filtereinrichtung, beispielsweise einem Blutfilter, vorgeschaltet (stromauf). Erfindungsgemäß ist der zweite Abschnitt in einem Betriebszustand der Funktionseinrichtung, insbesondere in Bezug auf eine Strömungsrichtung des Fluids während des Betriebs der Funktionseinrichtung, beispielsweise während einer Behandlung, einer Filtereinrichtung, beispielsweise einem Blutfilter, nachgeschaltet (stromab).

In erfindungsgemäßen Ausführungsformen ist die Wärmeaustauscheinrichtung wenigstens mit dem ersten und mit dem zweiten Abschnitt des Fluidkreislaufs, insbesondere lösbar oder austauschbar verbindbar, verbunden, und/oder angeordnet.

In manchen erfindungsgemäßen, beispielhaften Ausführungsformen ist wenigstens ein Abschnitt der Wärmeaustauscheinrichtung mit wenigstens einem Abschnitt des Fluidkreislaufs integral ausgebildet.

In erfindungsgemäßen Ausführungsformen ist die Wärmeaustauscheinrichtung insbesondere, bezogen auf die Strömungsrichtung des Fluids in einer Richtung durch die Wärmeaustauscheinrichtung hindurch, wenigstens in einem Abschnitt des Fluidkreislaufs vor wenigstens einem Durchflusssensor im Fluidkreislauf (d. h. stromauf zu diesem) angeordnet und/oder ausgebildet.

In manchen erfindungsgemäßen, beispielhaften Ausführungsformen weist der Fluidkreislauf und/oder die Wärmeaustauscheinrichtung wenigstens einen Heizer auf.

In bestimmten erfindungsgemäßen, beispielhaften Ausführungsformen ist ein Heizer, insbesondere bezogen auf die Strömungsrichtung des Fluids im Betrieb, vor, an oder nach der Wärmeaustauscheinrichtung, einem Durchflusssensor, einer Filtereinrichtung angeordnet.

In einigen erfindungsgemäßen, beispielhaften Ausführungsformen weist die Wärmeaustauscheinrichtung wenigstens einen, insbesondere massiven, Körper, beispielsweise ein Hartteil, auf.

In manchen erfindungsgemäßen, beispielhaften Ausführungsformen verlaufen im massiven Körper wenigstens ein Kanal, wenigstens ein Abschnitt des Fluidkreislaufs und/oder wenigstens ein Abschnitt, insbesondere ein Messabschnitt, eines Durchflusssensors.

In einigen erfindungsgemäßen, beispielhaften Ausführungsformen weist die Wärmeaustauscheinrichtung, oder wenigstens ein Teil hiervon, wenigstens ein, insbesondere gut, wärmeleitfähiges Material auf, beispielsweise Metall oder eine Metalllegierung, sowie Keramik, keramikgefüllte Kunststoffe oder Kunststoff mit geringen Wandstärken, oder besteht aus einem solchen oder aus einer Kombination hiervon.

In manchen erfindungsgemäßen, beispielhaften Ausführungsformen verläuft wenigstens ein Abschnitt wenigstens eines Durchflusssensors und/oder wenigstens ein Abschnitt des Fluidkreislaufs, insbesondere ein erster und/oder ein zweiter Abschnitt, beispielsweise ein Messabschnitt eines Durchflusssensors, durch wenigstens einen Abschnitt des wärmeleitenden Körpers der Wärmeaustauscheinrichtung, oder liegt hierin vor.

In manchen erfindungsgemäßen, beispielhaften Ausführungsformen ist wenigstens ein Abschnitt eines Durchflusssensors und/oder wenigstens ein Abschnitt des Fluidkreislaufs als eingelassener Kanal, vorzugsweise ein im Querschnitt geschlossener Kanal oder nicht geschlossener Kanal, etwa ein Halb- oder ein Teilkanal, in der Funktionseinrichtung und/oder in wenigstens einem wärmeleitenden Körper hiervon ausgebildet.

In einigen erfindungsgemäßen, beispielhaften Ausführungsformen kann wenigstens ein Kanal oder Teilkanal von einer Schicht, insbesondere von einer wärmeleitfähigen und/oder fluiddichten Schicht, beispielsweise eine Folie und/oder eine dünne Platte, nach außen und/oder mittels eines oder gegenüber einem weiteren Kanal abgedichtet sein.

In manchen erfindungsgemäßen, beispielhaften Ausführungsformen können zwei, insbesondere wärmeleitende, Körper, beispielsweise Hartteile, miteinander, vorzugsweise mittels wenigstens wärmeleitfähiger Mittel, beispielsweise einer wärmeleitfähigen Paste und/oder Platte, miteinander verbunden oder verbindbar sein, insbesondere derart, dass sie wieder voneinander lösbar sind.

In einigen erfindungsgemäßen, beispielhaften Ausführungsformen ist die Wärmeaustauscheinrichtung als separate, lösbare, austauschbare und/oder auswechselbare Einrichtung und/oder als Einwegartikel oder Disposable, beispielsweise als Kassette, oder als Teil hiervon, ausgebildet.

Erfindungsgemäß umfasst die Funktionseinrichtung ferner wenigstens einen Durchflusssensor. Alternativ oder ergänzend ist sie konfiguriert und/oder ausgebildet, um mit einem Durchflusssensor, insbesondere lösbar, verbunden und/oder verbindbar zu sein.

In erfindungsgemäßen Ausführungsformen ist der Durchflusssensor zum Messen wenigstens eines Volumenstroms im zweiten Abschnitt des Fluidkreislaufs ausgebildet und/oder angeordnet.

In manchen erfindungsgemäßen, beispielhaften Ausführungsformen sind die Funktionseinrichtung und/oder wenigstens ein Abschnitt des Fluidkreislaufs vorgesehen und/oder ausgebildet, um mit wenigstens einem Abschnitt wenigstens eines Durchflusssensors, beispielsweise einem Messabschnitt, vorzugsweise lösbar, beispielsweise austauschbar, verbindbar und/oder verbunden zu sein und/oder verbunden zu werden. In bestimmten erfindungsgemäßen, beispielhaften Ausführungsformen liegt eine solche Verbindung vor.

In einigen erfindungsgemäßen, beispielhaften Ausführungsformen ist wenigstens ein Abschnitt des Durchflusssensors, vorzugsweise wenigstens ein Messabschnitt hiervon, in der Funktionseinrichtung und/oder im Fluidkreislauf oder in wenigstens einem Abschnitt hiervon, integriert und/oder integral ausgebildet.

In manchen erfindungsgemäßen, beispielhaften Ausführungsformen ist der Durchflusssensor zum Erfassen wenigstens eines Volumenstroms jeweils in oder an wenigstens zwei, vorzugsweise unterschiedlichen, Abschnitten des Fluidkreislaufs angeordnet und/oder ausgebildet.

In erfindungsgemäßen Ausführungsformen ist wenigstens ein Durchflusssensor in der Wärmaustauschvorrichtung angeordnet und/oder ausgebildet, und/oder er ist Teil der Wärmaustauschvorrichtung.

In anderen erfindungsgemäßen, beispielhaften Ausführungsformen weist die Funktionseinrichtung nur einen Durchflusssensor auf. Beispielsweise kann ein Durchflusssensor Teil einer weiteren Anordnung oder Einrichtung, beispielsweise einer Bilanzierungseinrichtung oder einer Behandlungsvorrichtung, insbesondere einer erfindungsgemäßen sein.

In einigen erfindungsgemäßen, beispielhaften Ausführungsformen ist der Durchflusssensor oder wenigstens ein Abschnitt hiervon ein elektromagnetischer Durchflusssensor, insbesondere ein magnetisch-induktiver Durchflusssensor (im Folgenden auch kurz als "MID-Durchflusssensor" bezeichnet) oder weist wenigstens einen solchen auf.

In manchen erfindungsgemäßen, beispielhaften Ausführungsformen ist wenigstens ein Durchflusssensor ein wenigstens zweikanaliger Durchflusssensor oder weist einen solchen auf.

In einigen erfindungsgemäßen, beispielhaften Ausführungsformen sind die Funktionseinrichtung und/oder eine erfindungsgemäße Bilanzierungseinrichtung als Schlauchsystem, Schlauchset, Kassette, insbesondere als Blutkassette, ausgebildet.

In manchen erfindungsgemäßen, beispielhaften Ausführungsformen sind die Funktionseinrichtung und/oder die erfindungsgemäße Bilanzierungseinrichtung als Einwegartikel oder Disposable ausgebildet.

Unter dem Begriff "Einwegartikel oder Disposable", wie er hierhin verwendet wird, kann verstanden werden, dass die medizinische Funktionseinrichtung zur einmaligen Verwendung, beispielsweise in einem Verfahren zum extrakorporalen Behandeln des Bluts eines Patienten, vorgesehen ist. Sie kann als Disposable, als Einwegartikel, als Wegwerfartikel oder dergleichen vorgesehen und/oder vermarktet sein.

In manchen erfindungsgemäßen, beispielhaften Ausführungsformen weist die Funktionseinrichtung oder die Bilanzierungseinrichtung wenigstens eine Auswerteeinheit oder Auswerteeinrichtung zum Bestimmen wenigstens einer Fluidbilanz zwischen wenigstens zwei Abschnitten des Fluidkreislaufs auf, oder ist hiermit in Daten- oder Signalkommunikation verbunden. In einigen erfindungsgemäßen, beispielhaften Ausführungsformen weist die Funktionseinrichtung und/oder die erfindungsgemäße Bilanzierungseinrichtung wenigstens eine Steuer- und/oder Regelvorrichtung (welche im Folgenden vereinfachend als Steuervorrichtung bezeichnet wird, obgleich sie ebenso gut eine Regelvorrichtung sein kann) zum Steuern und/oder Regeln wenigstens eines Volumenstroms in wenigstens einem Abschnitt des Fluidkreislaufs auf.

In gewissen erfindungsgemäßen, beispielhaften Ausführungsformen ist die Steuervorrichtung als Regelvorrichtung ausgestaltet.

In manchen erfindungsgemäßen, beispielhaften Ausführungsformen kann der von einer Steuereinrichtung gesteuerte Volumenstrom insbesondere ein Volumenstrom eines weiteren Abschnitts des Fluidkreislaufs sein, beispielsweise der Volumenstrom, der durch eine Filtereinrichtung strömt. Der Volumenstrom kann beispielsweise eine Ultrafiltrationsrate sein.

In einigen erfindungsgemäßen, beispielhaften Ausführungsformen erfolgt die Regelung und/oder Steuerung des Volumenstroms wenigstens auf Basis und/oder wenigstens unter Auswertung der Fluidbilanz.

In manchen erfindungsgemäßen, beispielhaften Ausführungsformen bilden wenigstens die Auswerteeinrichtung und die Steuereinrichtung eine Einheit.

In einigen erfindungsgemäßen, beispielhaften Ausführungsformen stehen die Auswerteeinrichtung und/oder die Steuereinrichtung wenigstens in Datenverbindung miteinander und/oder mit wenigstens einem Durchflusssensor und/oder mit wenigstens einer Behandlungsvorrichtung und/oder mit jeweiligen wenigstens Abschnitten hiervon.

In einigen erfindungsgemäßen, beispielhaften Ausführungsformen findet das Bilanzieren in einer medizinischen Behandlungsvorrichtung, insbesondere in einer Blutbehandlungsvorrichtung, oder während einer medizinischen Behandlung, insbesondere einer Blutbehandlung statt. In einigen erfindungsgemäßen, beispielhaften Ausführungsformen nicht.

Unter dem "wenigstens einem Volumenstrom" kann hierhin beispielsweise der erste und/oder der zweite Volumenstrom, also der Volumenstrom des ersten und/oder des zweiten Abschnitts, verstanden werden.

In manchen erfindungsgemäßen, beispielhaften Ausführungsformen kann die Fluidbilanz (hier als FB abgekürzt), als Signal und/oder zur Erzeugung eines Signals für eine Steuer- und/oder eine Regelvorrichtung zum Steuern und/oder Regeln wenigstens eines Volumenstroms des Fluidkreislaufs, einer Pumprate wenigstens einer Pumpe des Fluidkreislaufs, eines Querschnitts wenigstens eines Abschnitts des Fluidkreislaufs verwendet werden.

In einigen erfindungsgemäßen, beispielhaften Ausführungsformen kann eine Fluidbilanz (FB) wenigstens aus einem ersten Volumenstrom (F1) und einem zweiten Volumenstrom (F2) gebildet werden.

In manchen erfindungsgemäßen, beispielhaften Ausführungsformen kann die Fluidbilanz (FB) folgendermaßen gebildet werden:

$$(\text{Formel 1}): \quad FB = F1 - F2,$$

oder

$$(\text{Formel 1'}): \quad FB = F2 - F1.$$

In einigen erfindungsgemäßen, beispielhaften Ausführungsformen wird die Fluidbilanz (FB) als Signal und/oder zur Erzeugung eines Signals für die Steuereinrichtung zum Steuern oder Regeln wenigstens eines Volumenstroms des Fluidkreislaufs verwendet. In manchen erfindungsgemäßen, beispielhaften Ausführungsformen können hierzu insbesondere der erste oder der zweite Volumenstrom erhöht oder erniedrigt werden.

Eine Korrektur kann in manchen erfindungsgemäßen, beispielhaften Ausführungsformen direkt auf elektronische Messwerte aufsetzen. Hierzu werden die aus den erhaltenen Messwerten zu bildenden Signale geeignet korrigiert.

In bestimmten erfindungsgemäßen, beispielhaften Ausführungsformen steht die Steuer- und/oder Regelungseinrichtung der erfindungsgemäßen Behandlungsvorrichtung in Signalverbindung mit der erfindungsgemäßen Bilanzierungseinrichtung. Sie ist konfiguriert und/oder programmiert zur Durchführung wenigstens einer Ausführungsform des erfindungsgemäßen Verfahrens, insbesondere im Zusammenwirken mit den hierzu jeweils erforderlichen Einrichtungen, beispielsweise wie hierin beschrieben.

In manchen erfindungsgemäßen, beispielhaften Ausführungsformen wird ein Volumenstrom durch eine Veränderung und/oder Anpassung einer Pumprate wenigstens einer Pumpe, insbesondere des Fluidkreislaufs oder eines hiermit verbundenen Kreislaufs, eines Querschnitts wenigstens eines Abschnitts im Fluidkreislauf, oder in einem verbundenen Kreislauf, gesteuert und/oder geregelt.

In bestimmten erfindungsgemäßen, beispielhaften Ausführungsformen ist die Bilanzierungseinrichtung ausgebildet und/oder konfiguriert, um mit wenigstens einem Durchflusssensor verbindbar zu sein, weist wenigstens einen solchen auf, oder ist mit wenigstens einem solchen verbunden. Der Durchflusssensor kann dabei zum Messen wenigstens eines Volumenstroms in wenigstens dem ersten und/oder dem zweiten Abschnitt des Fluidkreislaufs ausgebildet und/oder angeordnet sein.

In manchen erfindungsgemäßen, beispielhaften Ausführungsformen weist wenigstens die Bilanzierungseinrichtung, die Funktionseinrichtung oder die erfindungsgemäße Behandlungsvorrichtung wenigstens eine Auswerteeinheit zum Bilden wenigstens einer Fluidbilanz, insbesondere zwischen wenigstens zwei Volumenströmen in unterschiedlichen Abschnitten eines Fluidkreislaufs auf.

In einigen erfindungsgemäßen, beispielhaften Ausführungsformen weist wenigstens die Bilanzierungseinrichtung, die Funktionseinrichtung oder eine erfindungsgemäße Behandlungsvorrichtung wenigstens eine Auswerteeinrichtung zum Bestimmen wenigstens einer Fluidbilanz zwischen wenigstens zwei Abschnitten des Fluidkreislaufs auf.

In manchen erfindungsgemäßen, beispielhaften Ausführungsformen weist wenigstens die Bilanzierungseinrichtung, die Funktionseinrichtung oder eine erfindungsgemäße Behandlungsvorrichtung wenigstens eine Steuer- und/oder Regelvorrichtung zum Steuern und/oder Regeln wenigstens eines, insbesondere weiteren, Volumenstroms des Fluidkreislaufs auf.

In einigen erfindungsgemäßen, beispielhaften Ausführungsformen sind wenigstens die Auswerteeinrichtung und/oder die Steuereinrichtung nicht auf der Funktionseinrichtung ausgebildet oder angeordnet. Beispielsweise sind die Auswerteeinrichtung und/oder die Steuereinrichtung auf einer Behandlungsvorrichtung angeordnet oder ausgebildet.

Die erfindungsgemäße medizinische Behandlungsvorrichtung ist in bestimmten erfindungsgemäßen Ausführungsformen eine Blutbehandlungsvorrichtung, insbesondere eine Blutreinigungsvorrichtung wie eine Hämodialysiervorrichtung, eine Hämofiltrationsvorrichtung, eine Hämodiafiltrationsvorrichtung, allgemein eine DialyseVorrichtung, beispielsweise eine Vorrichtung zur Akutdialyse, zur Heimdialyse oder zur Peritonealdialyse. Sie kann eine Vorrichtung zum Durchführen von Leberersatzverfahren, eine Vorrichtung zum Durchführen von Immunadsorption oder dergleichen sein.

In bestimmten erfindungsgemäßen, beispielhaften Ausführungsformen wird als Verfahrensschritt wenigstens ein Volumenstrom in einem Abschnitt des Fluidkreislaufs, beispielsweise ein erster Volumenstrom in einem ersten Abschnitt, insbesondere vor Bildung der Fluidbilanz, eingestellt.

In manchen erfindungsgemäßen, beispielhaften Ausführungsformen werden wenigstens die Werte des eingestellten Volumenstroms und/oder einer eingestellten und/oder erfassten Temperatur erfasst, zumindest zeitweise gespeichert, und/oder hinterlegt. Manche, alle oder nur einige der jeweiligen Steuer- und/oder Regelvorrichtungen der Behandlungsvorrichtung, der Bilanzierungseinrichtung und/oder der Funktionseinrichtung können jeweils erfindungsgemäß entsprechende Vorrichtungen, insbesondere zum Durchführen eines erfindungsgemäßen Verfahrens und/oder zum Steuern und/oder Regeln, wie beispielsweise Blutpumpen, Ultrafiltrationspumpen oder dergleichen, aufweisen. Die entsprechenden Regel- und/oder Steuervorrichtungen können alternativ oder zusätzlich mit diesen Vorrichtungen jeweils wenigstens in Signalverbindung stehen.

In bestimmten erfindungsgemäßen, beispielhaften Ausführungsformen können die erfindungsgemäße Bilanzierungseinrichtung und/oder die medizinische Behandlungsvorrichtung ausgebildet und/oder konfiguriert sein, um das nicht beanspruchte Verfahren auszuführen. In manchen erfindungsgemäßen, beispielhaften Ausführungsformen wird das Verfahren im Zusammenwirken mit einer erfindungsgemäßen Funktionseinrichtung, einer erfindungsgemäßen Bilanzierungseinrichtung und/oder einer erfindungsgemäßen Blutbehandlungsvorrichtung ausgeführt.

In einigen erfindungsgemäßen, beispielhaften Ausführungsformen weist die Funktionseinrichtung, die Bilanzierungseinrichtung und/oder die Behandlungsvorrichtung keine Waage und/oder keine Bilanzkammer, insbesondere keine nicht-ideale, starre, Bilanzkammer, auf.

In bestimmten erfindungsgemäßen, beispielhaften Ausführungsformen weist das Verfahren keine gravimetrische Überwachung auf, und/oder die Ultrafiltrationsrate wird nicht direkt mit einem Sensor gemessen, insbesondere nicht mit einem MID-Sensor.

In einigen erfindungsgemäßen, beispielhaften Ausführungsformen weist die Funktionseinrichtung keinen Blutbehälter zur extrakorporalen temporären Speicherung auf, vorzugsweise keinen Blutbehälter, der ein zylinderförmiges Gehäuse aufweist.

In manchen erfindungsgemäßen, beispielhaften Ausführungsformen weist die Funktionseinrichtung keinen Luftabscheider auf.

In einigen erfindungsgemäßen, beispielhaften Ausführungsformen weist die Funktionseinrichtung kein Temperierelement, das in den Luftabscheider integrierbar ist, auf.

In manchen erfindungsgemäßen, beispielhaften Ausführungsformen weist die Funktionseinrichtung kein Peltier-Element auf.

In einigen erfindungsgemäßen, beispielhaften Ausführungsformen weist die Funktionseinrichtung keine Fördereinrichtung oder Fördereinrichtungen auf, insbesondere keine Pumpkammern.

In manchen erfindungsgemäßen, beispielhaften Ausführungsformen weist die Funktionseinrichtung keinen Durchlauferwärmer, insbesondere keine Mehrzahl von Durchlauferwärmern, auf.

In einigen erfindungsgemäßen, beispielhaften Ausführungsformen weist die Funktionseinrichtung kein Temperatur-Regelsystem auf, insbesondere keinen elektronischen Regler zum Regeln der Temperatur.

Manche oder alle erfindungsgemäßen Ausführungsformen können einen, mehrere oder alle der oben und/oder im Folgenden genannten Vorteile aufweisen.

Alle mit dem erfindungsgemäßen Verfahren erzielbaren Vorteile lassen sich in bestimmten erfindungsgemäßen Ausführungsformen ungeschmälert auch mit den erfindungsgemäßen Vorrichtungen erzielen.

In manchen erfindungsgemäßen Ausführungsformen wird vorteilhaft eine präzise Bilanzierung ermöglicht.

Diese kann vorteilhafterweise zugleich platzsparend und günstig ablaufen.

Während einer extrakorporalen Blutbehandlung werden regelmäßig Heizer oder Heizungen eingesetzt, beispielsweise um Wärmeverluste des Bluts außerhalb des Körpers auszugleichen. Dies kann einen komplexen Temperaturverlauf der verwendeten Flüssigkeiten, insbesondere im extrakorporalen Blutkreislauf, aber auch im restlichen Flüssigkeitskreislauf, verursachen. Somit weisen die zur Bilanzierung herangezogenen zu- und abgeführten Fluide regelmäßig voneinander verschiedene Temperaturen auf. Da der Temperaturunterschied eine Auswirkung auf das Volumen des Fluids haben kann, kann die Temperatur nicht nur das Volumen, sondern auch die Bilanzierung beeinflussen. Diese wird bei fehlender Berücksichtigung der Wirkung der Temperatur auf das Volumen möglicherweise verfälscht.

Messungen mit MID-Durchflusssensoren sind Messungen eines Volumenstroms. Die Dichte des Fluids ist temperaturabhängig. Somit sind der Volumenstrom, sowie ein hierzu korrespondierender Massenstrom, temperaturabhängig. In einigen erfindungsgemäßen Ausführungsformen wird jedoch erreicht, dass die Bilanzierung vorteilhaft unabhängig von der Auswirkung der Temperatur auf das zu bilanzierende Fluid erfolgt.

Weiterhin werden in bestimmten erfindungsgemäßen Ausführungen vorteilhafterweise Fehler, die aus einer temperaturabhängigen geometrischen und/oder volumetrischen Veränderung der verwendeten Sensoren resultieren, vorteilhaft vermieden.

In einigen erfindungsgemäßen Ausführungsformen werden vorteilhaft temperaturabhängige Fehler, insbesondere Geometrie- und/oder Dichtefehler, kompensiert.

In bestimmten erfindungsgemäßen Ausführungsformen kann der durch Temperaturunterschiede verursachte Fehler bei der Bilanzierung wenigstens um den Faktor 10 oder mehr verringert werden.

In manchen erfindungsgemäßen, beispielhaften Ausführungsformen wird vorteilhaft die Messung der Ultrafiltrationsrate vermieden, wodurch insbesondere ein Messsensor für die Messung der Ultrafiltrationsrate entfallen kann.

In einigen erfindungsgemäßen Ausführungsformen, insbesondere bei Ausgestaltungen der Funktionseinrichtung, der Bilanzierungseinrichtung und/oder der Wärmeaustauscheinrichtung als Einwegartikel, kann ein Keimwachstum zwischen aufeinander folgenden Behandlungen unterbrochen werden. Ferner kann das Risiko einer Übertragung einer Infektion von einem Patienten auf den nächsten mit derselben Behandlungsvorrichtung behandelten Patienten weiter gesenkt werden, ohne dass dies besondere Maßnahmen bei der Desinfektion erfordern würde.

In bestimmten erfindungsgemäßen Ausführungsformen kann vorteilhaft auf wenigstens einen Temperatursensor oder auf alle Temperatursensoren verzichtet werden. Ebenso kann das

erfindungsgemäße Verfahren mit einem oder ohne jeden Temperatursensor ausgeführt werden.

In manchen erfindungsgemäßen Ausführungsformen kann vorteilhaft auf wenigstens einen Heizer oder auf alle Heizer verzichtet werden. Ebenso kann das erfindungsgemäße Verfahren mit einem, mehreren oder ohne jeden Heizer ausgeführt werden.

In den Figuren bezeichnen identische Bezugszeichen gleiche, identische oder ähnliche Komponenten. Es gilt:

**Fig. 1** zeigt in vereinfachter Darstellung einen Fluidkreislauf einer erfindungsgemäßen Behandlungsvorrichtung;

**Fig. 2** zeigt in stark vereinfachter Darstellung eine erste exemplarische erfindungsgemäße Funktionseinrichtung mit zwei Durchflusssensoren;

**Fig. 3** zeigt in stark vereinfachter Darstellung eine exemplarische Anordnung einer Behandlungsvorrichtung mit einer weiteren erfindungsgemäßen Funktionseinrichtung;

**Fig. 4** zeigt in stark vereinfachter Darstellung eine weitere erfindungsgemäße Funktionseinrichtung;

**Fig. 5** zeigt in stark vereinfachter Darstellung eine weitere erfindungsgemäße Funktionseinrichtung; und

**Fig. 6** zeigt in stark vereinfachter Darstellung eine wiederum weitere erfindungsgemäße Funktionseinrichtung.

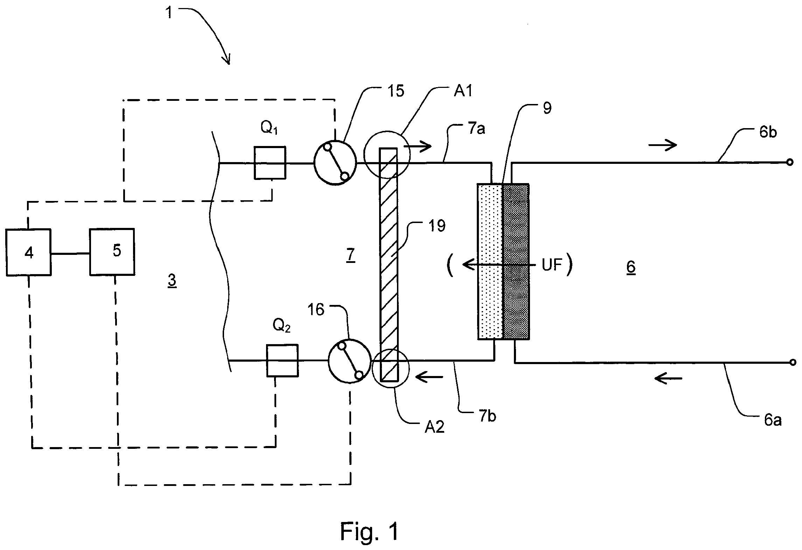

**Fig. 1** zeigt in stark vereinfachter Darstellung einen Abschnitt eines Fluidkreislaufs 1, welcher mit einer nur angedeuteten erfindungsgemäßen Behandlungsvorrichtung 3 in Fluidverbindung verbunden ist oder zumindest abschnittsweise Teil der Behandlungsvorrichtung ist. Die Behandlungsvorrichtung 3 ist mit einer erfindungsgemäßen Bilanzierungseinrichtung 4 verbunden oder weist diese auf. Die Behandlungsvorrichtung 3 oder die Bilanzierungseinrichtung 4 ist/sind zudem mit einer Steuer- oder Regelvorrichtung 5 zum Steuern oder Regeln von Pumpen, Ventilen, usw. zum Durchführen der Behandlung des Patienten verbunden oder weist diese auf. Die Bilanzierungseinrichtung 4 ist ferner mit zwei Durchflusssensoren Q1, Q2 oder mit wenigstens einem zweikanaligen Durchflusssensor verbunden oder weist diese auf. Im dargestellten Beispiel sind die Durchflusssensoren nicht als Teil der Behandlungsvorrichtung 3 dargestellt. Alternativ können die oder wenigstens ein Durchflusssensor als Teil der Behandlungsvorrichtung ausgebildet sein oder in oder an dieser angeordnet sein.

Die Bilanziereinrichtung 4 kann optional die Steuer- oder Regelvorrichtung 5 umfassen, oder mit ersterer in Signalverbindung stehen, wie beispielsweise in Fig. 1 gezeigt.

Die in Fig. 1 und allen folgenden Figuren dargestellte Behandlungsvorrichtung 3 ist rein exemplarisch als Ultrafiltrationsvorrichtung ausgestaltet, der Fluidkreislauf 1 als Kreislauf für die extrakorporale Blutbehandlung. Beide dieser Annahmen dienen allein dem besseren Verständnis der vorliegenden Erfindung anhand eines konkreten Beispiels und sollen die Erfindung keinesfalls beschränken.

Der Fluidkreislauf 1 weist einen extrakorporalen Blutkreislauf 6 und einen Dialysatkreislauf 7 auf, welche mittels einer Filtereinrichtung 9 rein exemplarisch sowohl im Stoff- als auch im Fluidaustausch stehen. Die Pfeile bezeichnen eine Strömungsrichtung der verschiedenen Flüssigkeiten während einer Behandlung eines nicht gezeigten Patienten mittels der Behandlungsvorrichtung 3.

Im extrakorporalen Blutkreislauf 6 fließt Blut des Patienten durch eine arterielle Blutschlauchleitung 6a zur Filtereinrichtung 9, um darin gereinigt zu werden. Durch eine venöse Blutschlauchleitung 6b wird das gereinigte Blut zum Patienten zurückgeführt. Die Blutschlauchleitungen 6a, 6b können entsprechende Schlauchklemmen aufweisen.

Im Dialysatkreislauf 7, welcher ein Substituat- oder Dialysatkreislauf sein kann und eine Dialysierflüssigkeitsleitung 7a und eine Dialysatleitung 7b aufweist, können vereinfachend eine erste Pumpe 15 und eine zweite Pumpe 16 zum Erzeugen eines bestimmten Volumenstroms innerhalb des Dialysatkreislaufs 7 angeordnet sein. Die Pumpen 15, 16 stehen, wie mit der Strichlinie angedeutet, mit der Steuer- oder Regelvorrichtung 5 optional jeweils in Signalkommunikation.

Der Dialysatkreislauf 7 weist ferner eine Wärmeaustauscheinrichtung 19, welche zwischen einem ersten Abschnitt A1 und einem zweiten Abschnitt A2 des Fluidkreislaufs 1 angeordnet ist und einen Wärmeausgleich beider Abschnitte ermöglicht und verursacht.

Auch der Blutkreislauf 6 kann optional wenigstens eine in den Figuren nicht gezeigte Pumpe zum Fördern von Blut aufweisen, welche beispielsweise ebenfalls mit der Steuer- oder Regelvorrichtung 5 in Signalkommunikation steht.

In Fig. 1 zeigt ein Pfeil eine rein optional erfolgende Ultrafiltration an, bei welcher Fluid aus dem Blutkreislauf 6 mit einem Durchfluss oder Volumenstrom UF (Ultrafiltrationsrate) über den Filter 9 in den Dialysatkreislauf 7 eintritt. Die Ultrafiltrationsrate UF kann mittels der Steuer- oder Regelvorrichtung 5 und entsprechender Pumpen, etwa der ersten, der zweiten Pumpe 15, 16, und/oder einer nicht dargestellten Blutpumpe, eingestellt bzw. gesteuert und/oder geregelt werden.

Die Anordnung der Fig. 1 ist aufgrund der entsprechend programmierten Bilanzierungseinrichtung 4 und/oder der entsprechend programmierten Steuer- und Regelvorrichtung 5 geeignet, das nicht beanspruchte Verfahren auszuführen.

Die in Fig. 1 gezeigten Pumpen 15, 16 und wenigstens die nicht dargestellte Blutpumpe sind der Einfachheit halber in den folgenden Figuren nicht erneut dargestellt. Sie können jedoch unverändert auch in den folgenden beschriebenen Ausführungsformen vorgesehen sein.

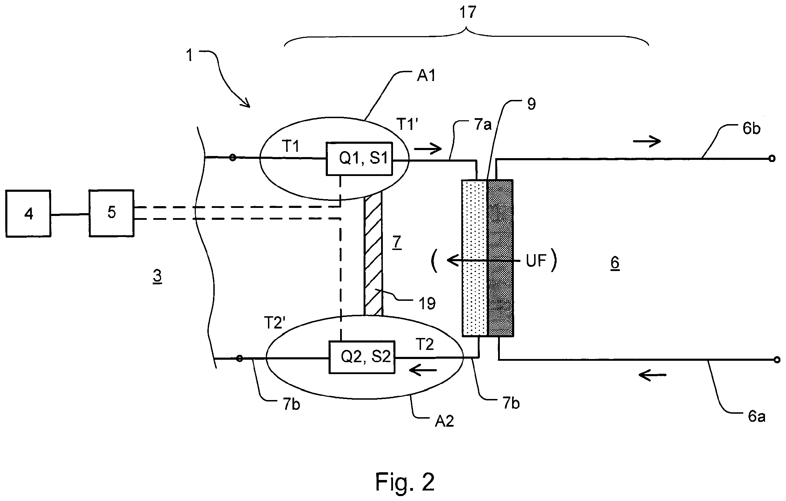

**Fig. 2** zeigt in stark vereinfachter Darstellung eine erfindungsgemäße Funktionseinrichtung 17, welche Teile des Dialysatkreislaufs 7 und rein optional auch Teile des Blutkreislaufs 6 umfasst. Die Funktionseinrichtung 17 weist üblicherweise nicht den Filter 9 auf; sie kann hiermit jedoch in Fluidkommunikation verbunden sein.

In einem ersten Abschnitt A1 des Dialysatkreislaufs 7 weist letzterer einen ersten Durchflusssensor Q1 auf oder ist hiermit verbunden. In einem zweiten Abschnitt A2 weist der Dialysatkreislauf 7 wenigstens einen zweiten Durchflusssensor Q2 auf oder ist hiermit verbunden. Der erste und/oder der zweite Durchflusssensor Q1, Q2 sind optional Teil der erfindungsgemäßen Funktionseinrichtung 17.

Der erste Abschnitt A1 liegt beispielsweise in der Dialysierflüssigkeitsleitung 7a, der zweite Abschnitt A2 beispielsweise in der Dialysatleitung 7b.

Die Temperatur des aus dem ersten Abschnitt A1, der sich optional stromauf der Filtereinrichtung 9 befindet, ausströmenden Fluids wird als T1' bezeichnet.

Die Temperatur des in den ersten Abschnitt A1 einströmenden Fluids wird als T1 bezeichnet.

Die Temperatur des Fluids, welches aus dem zweiten Abschnitt A2 ausströmt, wird als T2' bezeichnet. Der zweite Abschnitt A2 ist optional stromab der Filtereinrichtung 9 angeordnet.

Die Temperatur des Fluids, welches in den zweiten Abschnitt A2 einströmt, wird als T2 bezeichnet.

Die Temperatur T1 kann mittels eines rein optional vorgesehenen Temperatursensors S1 ermittelt werden. Ein Temperatursensor S1 ist, insbesondere bei Kenntnis oder Schätzung der Temperatur T1, jedoch nicht erforderlich. Der Temperatursensor S1 bzw. sein Einsatz sind vielmehr bei Kenntnis oder Schätzung der Temperatur T1 entbehrlich. Beispielsweise kann in einigen Behandlungsszenarien in ausreichend guter Näherung angenommen werden, dass T1 der Temperatur entspricht, mit welcher das Fluid, das den ersten Abschnitt A1 durchströmt, jener Temperatur entspricht, mit welcher das Fluid in einer Fluidquelle vorliegt.

Die Temperatur T2 kann mittels eines ebenfalls rein optional vorgesehenen Temperatursensors S2 ermittelt werden. Dieser Temperatursensor S2 ist, insbesondere bei Kenntnis oder Schätzung der Temperatur T2, jedoch nicht erforderlich. Tatsächlich zeigt beispielsweise gebrauchtes Dialysat beim Verlassen der Filtereinrichtung 9 eine geringe Temperaturschwankung. Regelmäßig bewegt sich die Temperatur der aus der Filtereinrichtung 9 ausströmenden Flüssigkeit im Bereich der Körpertemperatur (ca. 37°C). Beispielsweise kann daher in einigen Behandlungsszenarien in ausreichend guter Näherung angenommen werden, dass T2 der Körpertemperatur des Patienten entspricht.

Wie in Fig. 2 mittels Strichlinie angedeutet, stehen die Sensoren Q1, Q2 und gegebenenfalls S1 und/oder S2 vorzugsweise mit der Steuer- oder Regelvorrichtung 5, der Auswerteeinrichtung und/oder der Bilanzierungseinrichtung 4 in Signalverbindung.

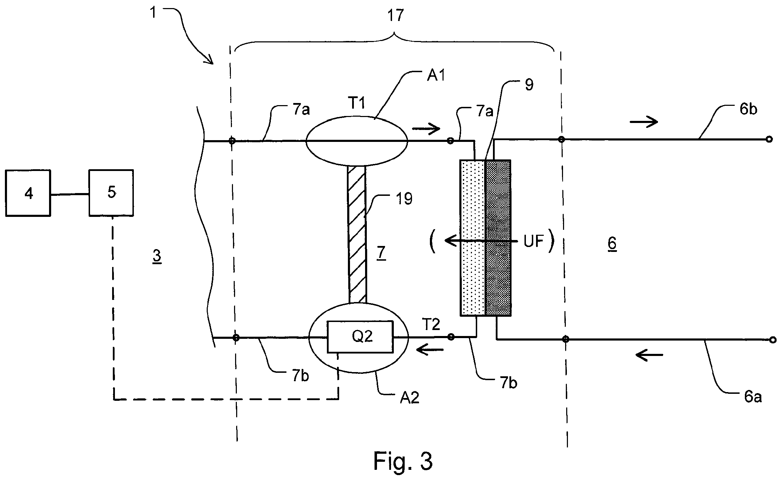

**Fig. 3,** die - wie auch die folgenden Figuren auf Fig. 1 - und Fig. 2 aufbaut, zeigt in stark vereinfachter Darstellung eine erfindungsgemäße Funktionseinrichtung 17 in einer weiteren beispielhaften Ausführungsform. Die Funktionseinrichtung 17 entspricht im Wesentlichen jener der Fig. 2.

Fig. 3 zeigt ferner, dass auch der erste Durchflusssensor Q1 entbehrlich sein kann. Dies gilt jedenfalls dann, wenn der erste Durchfluss F1 bekannt oder geschätzt werden kann. Dies kann beispielsweise der Fall sein, wenn wenigstens der erste Volumenstrom F1 oder der zweite Volumenstrom F2, insbesondere mittels einer hierfür geeigneten Vorrichtung, beispielsweise einer Pumpe und/oder einer Steuer- und/oder Regelvorrichtung und/oder einer Eingabevorrichtung, einstellbar und/oder eingestellt ist.

Das besondere Merkmal der Ausführungsform der Fig. 3, nach welchem es keines Durchflusssensors Q1 und/oder keines Temperatursensors S1 für den ersten Abschnitt A1 und/oder keines Temperatursensors S2 für den zweiten Abschnitt A2 bedarf, ist mit jeder anderen Ausgestaltung der vorliegenden Erfindung, wo sinnvoll, kombinierbar.

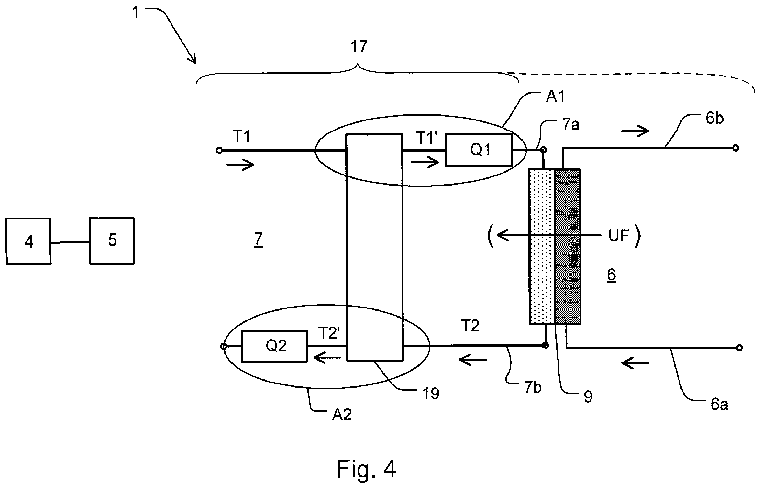

**Fig. 4** zeigt in stark vereinfachter Darstellung eine weitere erfindungsgemäße Ausführungsform der Funktionseinrichtung 17.

Die Funktionseinrichtung 17 weist optional einen ersten und einen zweiten Durchflusssensor Q1 und Q2 auf. Sie weist zwingend wenigstens eine Wärmeaustauscheinrichtung 19 auf oder ist hiermit verbunden.

Der optional vorgesehene erste Durchflusssensor Q1 ist in einem ersten Abschnitt A1 des Substituats- oder Dialysatkreislaufs 7 des Fluidkreislaufs 1 angeordnet, vorzugsweise in der Dialysierflüssigkeitsleitung 7a. Der optional vorgesehene zweite Durchflusssensor Q2 ist in einem zweiten Abschnitt A2 des Substituats- oder Dialysatkreislaufs 7 des Fluidkreislaufs 1 angeordnet, vorzugsweise in der Dialysatleitung 7b.

Der erste Abschnitt A1 liegt rein optional stromauf einer Filtereinrichtung 9 (beispielsweise eines Blutfilters), der zweite Abschnitt A2 liegt rein optional stromab der Filtereinrichtung 9. In diesem Beispiel stehen der erste und der zweite Abschnitt A1, A2 optional jeweils mit der Filtereinrichtung 9 in Fluidverbindung.

Die Wärmeaustauscheinrichtung 19 ist entlang der Strömungsrichtung des Fluids während der Blutbehandlung, welche mittels Pfeilen angedeutet ist, für einen Austausch von Wärme oder thermischer Energie zwischen dem Fluid, welches den ersten Abschnitt A1 durchströmt, und dem Fluid, welches den zweiten Abschnitt A2 durchströmt, angeordnet.

Insbesondere ist die Wärmeaustauscheinrichtung 19 optional stromauf des ersten Durchflusssensors Q1 angeordnet, welcher stromauf der Filtereinrichtung 9 angeordnet ist. Die Wärmeaustauscheinrichtung 19 ist zugleich optional stromauf des zweiten Durchflusssensors Q2 angeordnet, welcher stromab der Filtereinrichtung 9 angeordnet ist.

Die Wärmeaustauscheinrichtung 19 kann optional Teil des ersten und/oder des zweiten Abschnitt A1, A2 sein, insbesondere ein integraler Teil hiervon, und/oder optional mit dem ersten und/oder mit dem zweiten Abschnitt A1, A2 nur, insbesondere thermisch, verbunden sein.

Die Wärmeaustauscheinrichtung 19 ist vorgesehen, geeignet und/oder ausgebildet, um einen Wärmeaustausch zwischen dem Fluid, das den ersten Abschnitt A1 durchströmt, und dem Fluid, das den zweiten Abschnitt A2 durchströmt, zu bewirken.

Ein Wärmeausgleich kann bedeuten, dass die Differenz zwischen einer Temperatur T1' des Fluids nach Verlassen der Wärmaustauschvorrichtung 19, aber vor oder an dem ersten Durchflusssensor Q1, und insbesondere vor der Filtereinrichtung 9, und einer Temperatur T2' der ausströmenden Flüssigkeit nach dem Durchgang der Wärmaustauschvorrichtung 19, aber vor oder an dem zweiten Durchflusssensor Q2 Null oder im Wesentlichen Null ist. Es kann erfindungsgemäß auch zu verstehen sein, dass vorstehend genannte Differenz jedenfalls kleiner als die Differenz zwischen den Temperaturen T1 und T2 ist, welche das Fluid oder die Fluide jeweils vor Eintritt in die Wärmaustauschvorrichtung 19 aufwies.

Es kann erfindungsgemäß zusätzlich vorgesehen sein, die Temperatur T1 und/oder die Temperatur T2, beispielsweise mittels wenigstens eines Temperatursensors (nicht dargestellt), zu erfassen und/oder, insbesondere mittels einer geeigneten Einrichtung wie einem Heizer, einzustellen.

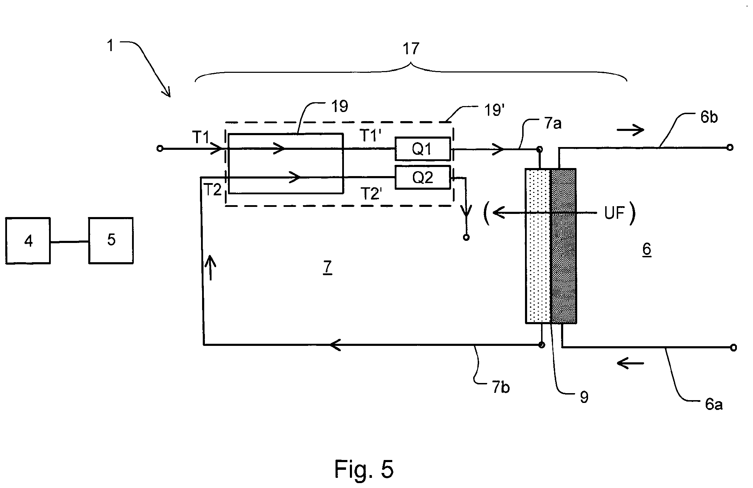

**Fig. 5** zeigt in stark vereinfachter Darstellung noch eine wiederum weitere beispielhafte Ausführungsform der erfindungsgemäßen Funktionseinrichtung.

Die Anordnung der Wärmeaustauscheinrichtung 19 und der Durchflusssensoren Q1 und Q2 entspricht grundsätzlich jener der Fig. 4. So sind beispielsweise die Durchflusssensoren Q1 und Q2 der Wärmeaustauscheinrichtung 19 bezüglich einer Strömungsrichtung des Fluids (einer medizinischen Flüssigkeit) wie in Fig. 4 jeweils nachgeschaltet.

Ebenso ist die Wärmeaustauscheinrichtung 19 vorgesehen und/oder angeordnet, um einen Wärmeausgleich zwischen den der Wärmeaustauscheinrichtung 19 jeweils - insbesondere unmittelbar - nachgelagerten Abschnitten zu bewirken.

Der mit dem Bezugszeichen 19' bezeichnete Bereich kann als von der Funktionseinrichtung 17 abnehmbarer oder lösbarer oder aus dieser herausnehmbarer Bereich oder Einsatz ausgebildet sein. Insbesondere umfasst dieser herausnehmbare Bereich 19' wenigstens die Wärmeaustauscheinrichtung 19. Zudem kann der herausnehmbare Bereich 19', rein optional, wenigstens einen oder beide Durchflusssensoren Q1, Q2, oder wenigstens Abschnitte hiervon, beispielsweise wie dargestellt, umfassen.

Der herausnehmbare Bereich 19' kann rein optional als Einwegartikel oder Disposable ausgebildet sein. Dasselbe kann auf die Funktionseinrichtung 17 in jeder erfindungsgemäßen Ausführungsform zutreffen.

Der herausnehmbare Bereich 19' kann rein optional wenigstens einen massiven, insbesondere gut wärmeleitenden Körper, beispielsweise ein metallisches Hartteil umfassen, in welchem die entsprechenden Abschnitte A1, A2 des Fluidkreislaufs 7 verlaufen.

Beide entsprechenden Abschnitte A1, A2 des Fluidkreislaufs 7 können exemplarisch innerhalb der Wärmeaustauscheinrichtung 19 durch eine dünne, insbesondere fluiddichte, Folie und/oder Platte voneinander getrennt sein.

Auch hier kann rein optional vorgesehen sein, nur einen Durchflusssensor Q1 oder Q2 zu verwenden, insbesondere wenn wenigstens einer der beiden Durchflüsse F1 und/oder F2, mit welchem das Fluid jeweils durch die Abschnitte A1, A2 strömt, bereits eingestellt und/oder einstellbar ist.

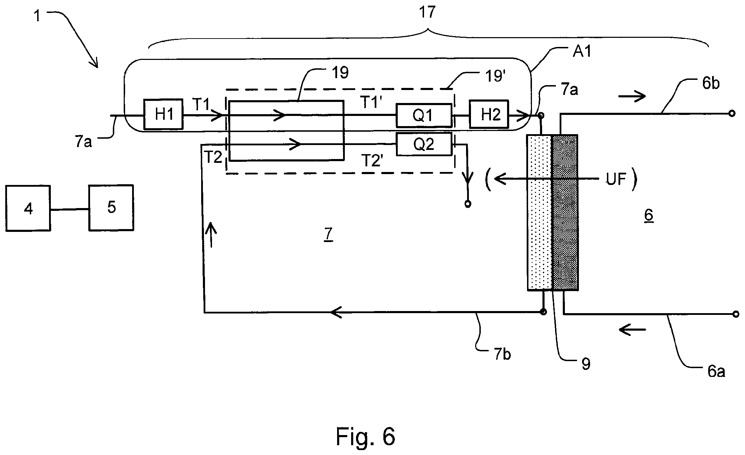

**Fig. 6** zeigt in stark vereinfachter Darstellung die Funktionseinrichtung 17 in einer wiederum weiteren erfindungsgemäßen Ausführungsform. Dabei kann die Anordnung und/oder Ausführung des herausnehmbaren Bereichs 19' der Fig. 5 oder ein anderer Abschnitt der Funktionseinrichtung 17 zusätzlich wenigstens einen Heizer Hi (i=1, 2) aufweisen.

Der Heizer Hi kann, insbesondere im ersten Abschnitt A1 des Fluidkreislaufs 1 angeordnet sein.

Im dargestellten Beispiel sind zwei Heizer H1 und H2 dargestellt. Beide sind beispielhaft im ersten Abschnitt A1 angeordnet und/oder ausgebildet. Beispielsweise ist der erste Heizer H1 dem herausnehmbaren Bereich 19' vorgelagert, während der zweite Heizer H2 beispielsweise dem herausnehmbaren Bereich 19' nachgelagert ist. Rein exemplarisch sind beide Heizer H1, H2 nicht Teil der Funktionseinrichtung 17. Sie könnten dies jedoch - zumindest abschnittsweise - sein. Rein exemplarisch sind beide Durchflusssensoren Q1, Q2 nicht Teil der Funktionseinrichtung 17. Sie könnten rein exemplarisch Teil der Bilanzierungseinrichtung 4 und/oder der Behandlungsvorrichtung 3 sein.

Es kann erfindungsgemäß vorgesehen sein, nur einen Heizer H1 oder H2 im Fluidkreislauf 7 vorzusehen.

Es kann auch vorgesehen sein, dass wenigstens ein Heizer Hi, insbesondere im ersten Abschnitt A1 des Fluidkreislaufs 7, der Wärmeaustauscheinrichtung 19 vorgelagert ist und/oder einem der Wärmeaustauscheinrichtung 19 nachgelagerten Durchflusssensor nachgelagert ist (nicht dargestellt).

In einer weiteren, nicht dargestellten erfindungsgemäßen Ausführungsform der Funktionseinrichtung 17 kann ein Heizer Hi rein optional an, in und/oder wenigstens in Verbindung mit der Wärmeaustauscheinrichtung 19, dem herausnehmbaren Bereich 19', oder wenigstens Abschnitten jeweils hiervon, ausgebildet und/oder angeordnet sein.

Der Heizer kann ausgebildet sein, um eine vorbestimmte minimale Temperatur des Fluids zu gewährleisten. Somit kann vorteilhaft in bestimmten Ausführungsformen vollständig auf eine Temperaturmessung verzichtet werden.

Heizer, wie sie in Fig. 6 gezeigt sind, können selbstredend auch mit den Anordnungen der in den vorangegangenen Figuren gezeigten Ausführungsformen kombiniert werden.

Mittels der Anordnung einer der Figuren und der besonderen Ausgestaltung der Bilanzierungseinrichtung 4 kann eine korrekte Fluidbilanz erzeugt werden. Alternativ oder ergänzend kann mittels der Steuer- und/oder Regelvorrichtung 5 eine Ultrafiltrationsrate angepasst werden.

### Bezugszeichenliste

| | |
|---|---|
| 1 | Fluidkreislauf |
| 3 | Behandlungsvorrichtung |
| 4 | Bilanzierungseinrichtung |
| 5 | Steuer- oder Regelvorrichtung |
| 6 | Blutkreislauf |
| 6a | arterielle Blutschlauchleitung |
| 6b | venöse Blutschlauchleitung |
| 7 | Dialysat- und/oder Substituatkreislauf |
| 7a | Dialysierflüssigkeitsleitung |
| 7b | Dialysatleitung |
| 9 | Filtereinrichtung |
| 15 | erste Pumpe |
| 16 | zweite Pumpe |
| 17 | Funktionseinrichtung |
| 19 | Wärmeaustauscheinrichtung herausnehmbarer Bereich der |
| 19' | Funktionseinrichtung erster Abschnitt des Dialysat- und/oder |
| A1 | Substituatkreislaufs zweiter Abschnitt des Dialysat- und/oder |
| A2 | Substituatkreislaufs |
| Q1 | erster Durchflusssensor |
| Q2 | zweiter Durchflusssensor |
| S1 | Temperatursensor |
| S2 | Temperatursensor |
| F1 | erster Volumenstrom im ersten Abschnitt des Fluidkreislaufs zweiter Volumenstrom im zweiten Abschnitt des |
| F2 | Fluidkreislaufs |
| Hi (i=1, 2, ...) | Heizer |

### Patentansprüche

1. Medizinische Funktionseinrichtung (17) mit wenigstens einem Fluidkreislauf (1) oder einem Abschnitt hiervon, wobei die Funktionseinrichtung (17) oder der Fluidkreislauf (1) wenigstens eine Wärmeaustauscheinrichtung (19) aufweist oder hiermit verbunden ist, welche zwischen einem ersten Abschnitt (A1) des Fluidkreislaufs (1) und einem zweiten Abschnitt (A2) des Fluidkreislaufs (1) angeordnet und/oder ausgebildet ist,
wobei der zweite Abschnitt (A2) stromab einer Filtereinrichtung (9) liegt,
wobei ein zweiter Durchflusssensor (Q2) im zweiten Abschnitt liegt und **dadurch gekennzeichnet, dass** der zweite Durchflusssensor (Q2)

     - in der Wärmeaustauschvorrichtung (19) angeordnet oder Teil der Wärmeaustauschvorrichtung (19) ist;
     oder
     - der Wärmeaustauscheinrichtung (19) bezüglich der Strömungsrichtung eines Fluids nachgeschaltet ist.

2. Funktionseinrichtung (17) nach Anspruch 1, wobei die Funktionseinrichtung einen ersten Durchflusssensor (Q1) im ersten Abschnitt (A1) aufweist.

3. Funktionseinrichtung (17) nach Anspruch 1 oder 2, wobei die Wärmeaustauscheinrichtung (19) derart dimensioniert, angeordnet, verbunden und/oder ausgebildet ist, dass die Temperatur (T1') des ersten Abschnitts (A1) des Fluidkreislaufs (1) und die Temperatur (T2') des zweiten Abschnitts (A2) des Fluidkreislaufs (1) ausgeglichen oder gleich sind oder dass die Temperaturdifferenz der in den betreffenden Abschnitten durchströmenden Fluide und/oder der jeweiligen Abschnitte vor und nach dem Verlassen der Wärmeaustauscheinrichtung wenigstens reduziert ist.

4. Funktionseinrichtung (17) nach einem der vorangehenden Ansprüche, wobei die Wärmeaustauscheinrichtung (19) wenigstens einen wärmeleitenden Körper umfasst, wobei wenigstens in einem Abschnitt des wärmeleitenden Körpers wenigstens ein Abschnitt des Fluidkreislaufs (1) verläuft oder angeordnet ist.

5. Funktionseinrichtung (17) nach einem der vorangehenden Ansprüche, wobei wenigstens die Wärmeaustauscheinrichtung (19) als separate, austauschbare und/oder von der Funktionseinrichtung lösbare Einrichtung und/oder als Einwegartikel ausgebildet ist.

6. Funktionseinrichtung (17) nach einem der vorangehenden Ansprüche, wobei der zweite Durchflusssensor (Q2) zum Messen wenigstens eines Volumenstroms (F2) in einem zweiten Abschnitt (A2) des Fluidkreislaufs (1) angeordnet ist.

7. Funktionseinrichtung (17) nach Anspruch 6, wobei der zweite Durchflusssensor (Q2) als magnetischinduktiver Durchflusssensor oder als ein Abschnitt hiervon ausgebildet ist.

8. Funktionseinrichtung (17) nach einem der Ansprüche 6 bis 7, wobei der Durchflusssensor (Q2) wenigstens als zweikanaliger Durchflusssensor ausgebildet ist.

9. Funktionseinrichtung (17) nach einem der vorangehenden Ansprüche, wobei die Funktionseinrichtung (17) als Schlauchsystem und/oder als Kassette ausgebildet ist.

10. Funktionseinrichtung (17) nach einem der vorangehenden Ansprüche, welche als Einwegartikel oder Disposable ausgebildet ist.

11. Bilanzierungseinrichtung (4) zum Bestimmen wenigstens einer Fluidbilanz zwischen wenigstens oder genau einem ersten Volumenstrom (F1) in einem ersten Abschnitt (A1) eines Fluidkreislaufs (1) und einem zweiten Volumenstrom (F2) in einem zweiten Abschnitt (A2) des Fluidkreislaufs (1), wobei die Bilanzierungseinrichtung (4) wenigstens eine Funktionseinrichtung (17) nach einem der Ansprüche 1 bis 10 aufweist und/oder wobei die Bilanzierungseinrichtung (4) wenigstens in Datenverbindung mit wenigstens einer solchen Funktionseinrichtung (17) oder Teilen hiervon steht.

12. Bilanzierungseinrichtung (4) nach Anspruch 11 umfassend wenigstens eine Auswerteeinheit zum Bilden wenigstens einer Fluidbilanz zwischen wenigstens einem oder genau einem ersten Volumenstrom (F1) in einem ersten Abschnitt (A1) eines Fluidkreislaufs (1) und einem zweiten Volumenstrom (F2) in einem zweiten Abschnitt (A2) des Fluidkreislaufs (1), und/oder eine Steuer- und/oder Regelvorrichtung (5) zum Steuern und/oder Regeln wenigstens eines weiteren Volumenstroms (F1, F2, UF) des Fluidkreislaufs (1).

13. Bilanzierungseinrichtung (4) nach Anspruch 12, wobei die Auswerteeinheit und/oder die Steuer- und/oder Regelvorrichtung (5) Teil einer Behandlungsvorrichtung (3) sind, oder an oder in einer solchen angeordnet sind.

14. Medizinische Behandlungsvorrichtung (3) zum Behandeln eines medizinischen Fluids mit wenigstens einer Bilanzierungseinrichtung (4) nach einem der Ansprüche 11 bis 13 und/oder mit wenigstens einer Funktionseinrichtung (17) nach einem der Ansprüche 1 bis 10.

15. Medizinische Behandlungsvorrichtung (3) nach Anspruch 14 umfassend eine Steuer- oder Regelvorrichtung konfiguriert zum Ausführen eines Verfahrens zum Bestimmen einer Fluidbilanz (FB) zwischen wenigstens einem ersten Volumenstrom (F1) eines ersten Abschnitts (A1) eines Fluidkreislaufs (1) einer medizinischen Funktionseinrichtung (17) und wenigstens einem zweiten Volumenstrom (F2) eines zweiten Abschnitts (A2) des Fluidkreislaufs (1) der medizinischen Funktionseinrichtung (17) und/oder zum Steuern und/oder Regeln wenigstens eines Volumenstroms (F1, F2, UF) eines Abschnitts des Fluidkreislaufs (1) der medizinischen Funktionseinrichtung (17), wobei die Behandlungsvorrichtung (3) eine Funktionseinrichtung (17) nach einem der Ansprüche 2 bis 9 mit einem ersten und einem zweiten Durchflusssensor (Q1, Q2) aufweist und wobei das Verfahren die folgenden Schritte umfasst:

- Erfassen oder Einstellen wenigstens eines ersten Volumenstroms (F1) in wenigstens einem ersten Abschnitt (A1) des Fluidkreislaufs (1);
- Erfassen wenigstens eines zweiten Volumenstroms (F2) in wenigstens einem zweiten Abschnitt (A2) des Fluidkreislaufs (1);
- Bildung einer Fluidbilanz (FB) aus wenigstens dem ersten Volumenstrom (F1) und dem zweiten Volumenstrom (F2); und
- wobei die Fluidbilanz (FB), zum Steuern und/oder Regeln wenigstens eines Volumenstroms (F1, F2, UF) des Fluidkreislaufs (1), einer Pumprate wenigstens einer Pumpe des Fluidkreislaufs (1), und/oder eines Querschnitts wenigstens eines Abschnitts des Fluidkreislaufs (1) verwendet wird.

**Claims**

1. A medical functional device (17) comprising at least one fluid circuit (1) or a section thereof, the functional device (17) or the fluid circuit (1) comprising or being connected with at least one heat exchange device (19) which is arranged and/or designed between a first section (A1) of the fluid circuit (1) and a second section (A2) of the fluid circuit (1),
wherein the second section (A2) is downstream of a filter device (9),
wherein a second flow sensor (Q2) is located in the second section and **characterized in that** the second flow sensor (Q2) :

- is arranged in the heat exchange device (19) or is part of the heat exchange device (19); or
- is connected downstream of the heat exchange device (19) with respect to the flow direction of a fluid.

2. The functional device (17) according to claim 1, wherein the functional device comprises a first flow sensor (Q1) in the first section (A1).

3. The functional device (17) according to claim 1 or 2, wherein the heat exchange device (19) is dimensioned, arranged, connected and/or designed such that the temperature (T1') of the first section (A1) of the fluid circuit (1) and the temperature (T2') of the second section (A2) of the fluid circuit (1) are balanced or equal, or that the temperature difference between the fluids flowing through the relevant sections and/or the respective sections is at least reduced before and after leaving the heat exchange device.

4. The functional device (17) according to anyone of the preceding claims, wherein the heat exchange device (19) comprises at least one heat conducting body, wherein at least one section of the fluid circuit (1) extends or is arranged in at least one section of the heat conducting body.

5. The functional device (17) according to anyone of the preceding claims, wherein at least the heat exchange device (19) is designed as a separate, exchangeable and/or detachable device from the functional device and/or as a single-use article.

6. The functional device (17) according to anyone of the preceding claims, wherein the second flow sensor (Q2) for measuring at least one volume flow (F2) is arranged in a second section (A2) of the fluid circuit (1).

7. The functional device (17) according to claim 6, wherein the second flow sensor (Q2) is designed as a magnetic-inductive flow sensor or as a section thereof.

8. The functional device (17) according to anyone of claims 6 to 7, wherein the flow sensor (Q2) is designed as at least a two-channel flow sensor.

9. The functional device (17) according to anyone of the preceding claims, wherein the functional device (17) is designed as a tubing system and/or as a cassette.

10. The functional device (17) according to anyone of the preceding claims, which is designed as a single-use article or as a disposable.

11. A balancing device (4) for determining at least one fluid balance between at least or exactly one first volume flow (F1) in a first section (A1) of a fluid circuit (1) and a second volume flow (F2) in a second section (A2) of the fluid circuit (1), the balancing device (4) comprising at least one functional device (17) according to anyone of claims 1 to 10 and/or the balancing device (4) being at least in data connection with at least one such functional device (17) or parts thereof.

12. The balancing device (4) according to claim 11 comprising at least one evaluation unit for forming at least one fluid balance between at least one or exactly one first volume flow (F1) in a first section (A1) of a fluid circuit (1) and a second volume flow (F2) in a second section (A2) of the fluid circuit (1), and/or a control and/or regulating apparatus (5) for controlling and/or regulating at least one further volume flow (F1, F2, UF) of the fluid circuit (1).

13. The balancing device (4) according to claim 12, wherein the evaluation unit and/or the control and/or regulating apparatus (5) is/are part of a treatment apparatus (3), or is/are arranged on or in such an apparatus.

14. A medical treatment apparatus (3) for treating a medical fluid with at least one balancing device (4) according to anyone of claims 11 to 13 and/or with at least one functional device (17) according to anyone of claims 1 to 10.

15. The medical treatment apparatus (3) according to claim 14 comprising a control or regulating apparatus configured to perform a method for determining a fluid balance (FB) between at least one first volume flow (F1) of a first section (A1) of a fluid circuit (1) of a medical functional device (17) and at least one second volume flow (F2) of a second section (A2) of the fluid circuit (1) of the medical functional device (17) and/or for controlling and/or regulating at least one volume flow (F1, F2, UF) of a section of the fluid circuit (1) of the medical functional device (17), wherein the treatment apparatus (3) comprises a functional device (17) according to anyone of claims 2 to 9 with a first and a second flow sensor (Q1, Q2) and wherein the method includes the following steps:

- detecting or adjusting at least one first volume flow (F1) in at least one first section (A1) of the fluid circuit (1) ;
- detecting at least one second volume flow (F2) in at least one second section (A2) of the fluid circuit (1)
- forming a fluid balance (FB) from at least the first volume flow (F1) and the second volume flow (F2); and
- wherein the fluid balance (FB) is used for controlling and/or regulating at least one volume flow (F1, F2, UF) of the fluid circuit (1), a pumping rate of at least one pump of the fluid circuit (1), and/or a cross section of at least one section of the fluid circuit (1).

**Revendications**

1. Un dispositif fonctionnel médical (17) comprenant au moins un circuit fluidique (1) ou une portion de celui-ci,
le dispositif fonctionnel (17) ou le circuit fluidique (1) comprenant ou étant relié à au moins un dispositif d'échange thermique (19) agencé et/ou conçu entre une première portion (A1) du circuit fluidique (1) et une seconde portion (A2) du circuit fluidique (1),
où la seconde portion (A2) est en aval d'un dispositif de filtration (9),
où un second débitmètre (Q2) est situé dans la seconde portion et **caractérisé en ce que** le second débitmètre (Q2):

- est agencé dans le dispositif d'échange thermique (19) ou fait partie du dispositif d'échange thermique (19); ou
- est relié en aval du dispositif d'échange thermique (19) par rapport au sens d'écoulement d'un fluide.

2. Le dispositif fonctionnel (17) selon la première revendication, où le dispositif fonctionnel comprend un premier débitmètre (Q1) dans la première portion (A1).

3. Le dispositif fonctionnel (17) selon la revendication 1 ou 2, où le dispositif d'échange thermique (19) est dimensionné, agencé, relié et/ou conçu de telle sorte que la température (T1') de la première portion (A1) du circuit fluidique (1) et la température (T2') de la seconde portion (A2) du circuit fluidique (1) soient équilibrées ou égales, ou que la différence de température entre les fluides traversant les portions concernées et/ou les portions respectives soit au moins réduite avant et après avoir quitté le dispositif d'échange thermique.

4. Le dispositif fonctionnel (17) selon l'une quelconque des revendications précédentes, où le dispositif d'échange thermique (19) comprend au moins un corps thermoconducteur, où au moins une partie du circuit fluidique (1) s'étend ou est agencée dans au moins une portion du corps thermoconducteur.

5. Le dispositif fonctionnel (17) selon l'une quelconque des revendications précédentes, où au moins le dispositif d'échange thermique (19) est conçu comme un dispositif séparé, interchangeable et/ou détachable du dispositif fonctionnel et/ou comme un article jetable.

6. Le dispositif fonctionnel (17) selon l'une quelconque des revendications précédentes, où le second débitmètre (Q2) pour mesurer au moins un débit volumique (F2) est agencé dans une seconde portion (A2) du circuit fluidique (1).

7. Le dispositif fonctionnel (17) selon la revendication 6, où le second débitmètre (Q2) est conçu comme un débitmètre magnéto-inductif ou comme une partie de celui-ci.

8. Le dispositif fonctionnel (17) selon l'une quelconque des revendications 6 à 7, où le débitmètre (Q2) est conçu au moins comme un débitmètre à deux canaux.

9. Le dispositif fonctionnel (17) selon l'une quelconque des revendications précédentes, où le dispositif fonctionnel (17) est conçu comme un système de tuyaux et/ou comme une cassette.

10. Le dispositif fonctionnel (17) selon l'une quelconque des revendications précédentes, conçu comme un article jetable ou à usage unique.

11. Un dispositif d'équilibrage (4) pour déterminer au moins un bilan fluidique entre au moins ou exactement un premier débit volumique (F1) dans une première portion (A1) d'un circuit fluidique (1) et un second débit volumique (F2) dans une seconde portion (A2) du circuit fluidique (1),
où le dispositif d'équilibrage (4) comprend au moins un dispositif fonctionnel (17) selon l'une quelconque des revendications 1 à 10 et/ou où le dispositif d'équilibrage (4) est au moins en connexion de données avec au moins un tel dispositif fonctionnel (17) ou des parties de celui-ci.

12. Le dispositif d'équilibrage (4) selon la revendication 11 comprenant au moins une unité d'évaluation pour former au moins un bilan fluidique entre au moins un ou exactement un premier débit volumique (F1) dans une première portion (A1) d'un circuit fluidique (1) et un second débit volumique (F2) dans une seconde portion (A2) du circuit fluidique (1), et/ou un appareil de commande et/ou de régulation (5) pour commander et/ou réguler au moins un débit volumique supplémentaire (F1, F2, UF) du circuit fluidique (1).

13. Le dispositif d'équilibrage (4) selon la revendication 12, où l'unité d'évaluation et/ou l'appareil de commande et/ou de régulation (5) fait/font partie d'un appareil de traitement (3), ou est/sont agencé(s) sur ou dans un tel dispositif.

14. Un appareil de traitement médical (3) pour traiter un fluide médical avec au moins un dispositif d'équilibrage (4) selon l'une quelconque des revendications 11 à 13 et/ou avec au moins un dispositif fonctionnel (17) selon l'une quelconque des revendications 1 à 10.

15. L'appareil de traitement médical (3) selon la revendication 14 comprenant un appareil de commande ou de régulation configuré pour exécuter un procédé de détermination d'un bilan fluidique (FB) entre au moins un premier débit volumique (F1) d'une première portion (A1) d'un circuit fluidique (1) d'un dispositif médical fonctionnel (17) et au moins un second débit volumique (F2) d'une seconde portion (A2) du circuit fluidique (1) du dispositif médical fonctionnel (17) et/ou configuré pour commander et/ou réguler au moins un débit volumique (F1, F2, UF) d'une portion du circuit fluidique (1) du dispositif fonctionnel (17),
   où l'appareil de traitement (3) comprend un dispositif fonctionnel (17) selon l'une des revendications 2 à 9 avec un premier et un second débitmètres (Q1, Q2) et où le procédé inclut les étapes suivantes:

   - la détection ou le réglage d'au moins un premier débit volumique (F1) dans au moins une première portion (A1) du circuit fluidique (1);
   - la détection d'au moins un second débit volumique (F2) dans au moins une seconde portion (A2) du circuit fluidique (1);
   - la formation d'un bilan fluidique (FB) à partir d'au moins le premier débit volumique (F1) et le second débit volumique (F2); et
   - où le bilan fluidique (FB) est utilisé pour commander et/ou réguler au moins un débit volumique (F1, F2, UF) du circuit fluidique (1), un débit de pompage d'au moins une pompe du circuit fluidique (1), et/ou une section transversale d'au moins une portion du circuit fluidique (1).

Fig. 1

Fig. 2

Fig. 3

Fig. 4

Fig. 5

Fig. 6

EP 3 274 012 B1

**IN DER BESCHREIBUNG AUFGEFÜHRTE DOKUMENTE**

**In der Beschreibung aufgeführte Patentdokumente**

- EP 0715859 A